# EUROPÄISCHE PATENTSCHRIFT

(11) **EP 2 863 891 B1**
(45) Veröffentlichungstag und Bekanntmachung des Hinweises auf die Patenterteilung: **26.08.2020**
(21) Anmeldenummer: 13730598.3
(22) Anmeldetag: 24.06.2013
(51) Int. Cl.: A61K 9/14, A61K 9/22, A61K 9/24

(54) **ARZNEIFORM ZUR VERLÄNGERTEN FREISETZUNG VON WIRKSTOFFEN**
PHARMACEUTICAL FORM FOR EXTENDED RELEASE OF ACTIVE SUBSTANCES
FORME GALÉNIQUE POUR LA LIBÉRATION PROLONGÉE DE SUBSTANCES ACTIVES

(30) Priorität: 25.06.2012 DE 102012105512
(43) Veröffentlichungstag der Anmeldung: 29.04.2015
(73) Patentinhaber: Hennig Arzneimittel GmbH&Co. Kg, 65439 Flörsheim am Main (DE)
(72) Erfinder: FRANCAS, Gernot, 67551 Worms (DE); PRZYKLENK, Karl-Heinz, 64521 Groß-Gerau (DE)
(74) Vertreter: Fuchs Patentanwälte Partnerschaft mbB
(86) Internationale Anmeldenummer: PCT/EP2013/063163
(87) Internationale Veröffentlichungsnummer: WO 2014/001268

(56) Entgegenhaltungen:
- EP-A2- 1 614 413
- WO-A1-2009/074609
- WO-A1-2010/142456
- WO-A1-2011/024029
- WO-A1-2012/175737
- WO-A1-2012/175747
- WO-A1-2013/030119
- WO-A2-2006/099865
- WO-A2-2007/086078
- DATABASE WPI Week 200828 Thomson Scientific, London, GB; AN 2008-D85368 XP002714651, & CN 101 049 309 A (CHEN X) 10. Oktober 2007 (2007-10-10)

## Beschreibung

Diese Erfindung betrifft Arzneiformen zur verlängerten Freisetzung von Wirkstoffen, insbesondere von Wirkstoffen, die bei hohen pH-Werten schlecht löslich sind. Eine besondere Ausführungsform betrifft eine solche Arzneiform, die nicht nur einen ersten Wirkstoff, der bei hohen pH-Werten schlecht löslich ist, verlängert freisetzt, sondern auch einen zweiten Wirkstoff verzögert abgibt, insbesondere einen zweiten Wirkstoff, der bei hohen pH-Werten eine bessere Löslichkeit als der erste Wirkstoff aufweist. Zu diesem Zweck weisen Ausführungsformen dieser Erfindung mehrere Kompartimente auf, die jeweils einen Wirkstoff enthalten können. Eine solche erfindungsgemäße Arzneiform kann beispielsweise als Schichttablette ausgestaltet sein.

Viele pharmazeutische Wirkstoffe sind organische Basen, d.h. sie weisen funktionelle Gruppen auf, die einen pK_{B}-Wert von weniger als 7, insbesondere weniger als 5, aufweisen. Solche Wirkstoffe lösen sich schlecht im Darmmilieu. Der Grund ist, dass im Darm ein alkalischer pH-Wert vorherrscht. Daher sind die organischen Basen dort nicht protoniert und somit ungeladen. Diese Moleküle sind dann schlecht löslich. Der Darm, insbesondere der Dünndarm, ist aber der Ort, an dem typischerweise die Resorption der Wirkstoffe stattfindet und stattfinden soll.

Damit ein Wirkstoff resorbiert werden kann, muss er sich zunächst lösen. Löst er sich nicht, wird er nicht aus seiner Arzneiform frei gesetzt.

Für die oben genannten organischen Basen gilt, dass sie sich typischerweise in saurem Milieu besser lösen. Der Grund ist, dass die basische Gruppe in dem jeweiligen Molekül in saurem Milieu protoniert ist, sodass die organische Base eine positive Ladung trägt. Ein solches saures Milieu herrscht typischerweise im nüchternen menschlichen Magen vor.

Die oben skizzierten Umstände einer besseren Löslichkeit im nüchternen Magen einerseits und einer schlechteren Löslichkeit im Darm andererseits führen zu Problemen bei der Formulierung von Arzneiformen, insbesondere wenn eine verlängerte Freisetzung erwünscht ist.

Es muss berücksichtigt werden, dass der pH-Wert im menschlichen Magen nicht immer sehr sauer ist. Nach der Einnahme einer Mahlzeit erreicht der pH-Wert durchaus neutrale Werte, im Allgemeinen Werte zwischen etwa pH 4 und 6. Erst nach und nach sinkt der pH-Wert wieder. Im Allgemeinen ist ein pH-Wert von < 2 erst nach 90 bis 120 Minuten wieder erreicht. Das bedeutet für den Wirkstoff mit den oben beschriebenen Eigenschaften, dass er, wenn er nach einer Mahlzeit eingenommen wird, zunächst eine schlechte Löslichkeit im Magen aufweist, die sich mit der Zeit verbessert, weil der pH-Wert im Magen wieder saurer wird.

Es ist besonders schwierig eine verlängerte Freisetzung solcher schlecht löslicher Wirkstoffe zu erzielen. Ein Ansatz, dem skizzierten Problem zu begegnen, ist die Formulierung von Arzneiformen mit verlängerter Freisetzung. Dies wird oft dadurch erzielt, dass die Arzneiform mit einem Überzug versehen wird, der den Wirkstoff nur langsam freigibt, oder dadurch, dass der Wirkstoff in einer Matrix dispergiert wird, die nur langsam erodiert.

Dabei tritt allerdings wieder das Problem zutage, dass sich der Wirkstoff im alkalischen Milieu des Darmes nur schlecht löst. Für die verlängerte, möglichst lineare Freisetzung ist es aber erforderlich, dass sich der Wirkstoff kontinuierlich löst. In der Praxis führt dies dazu, dass Arzneiformen, die einen basischen Wirkstoff mit schlechter Darmlöslichkeit verlängert freisetzen sollen, diesem Anspruch zwar zunächst (im Magen) gerecht werden, sobald die Arzneiform aber den Darm erreicht eine so schlechte Freisetzung erzielen, dass die Anwendung nicht zielführend ist.

Beispielsweise beschreibt CN 101049309 die Verbesserung der Bioverfügbarkeit eines bei basischem pH Wertes schlecht löslichen Wirkstoffes. Der Wirkstoff wird jedoch in einer Darreichungsform bereitgestellt, die sich unter der Zunge auflöst und den Wirkstoff freisetzt. Diese Darreichungsform führt zu einer schnellen Freisetzung in hohen Mengen, sodass die Absorption des Wirkstoffes über die Mundschleimhaut erfolgt und eine Aufnahme über den gastrointestinalen Weg vermieden wird.

Ein weiteres Problem tritt auf, wenn zwei Wirkstoffe mit unterschiedlichem Lösungsverhalten aus einer Arzneiform in gleicher Weise freigesetzt werden sollen. Bei besser löslichen Wirkstoffen, insbesondere solchen mit permanenter Ladung, tritt das Problem auf, dass diese besser löslichen Wirkstoffe nur verzögert freigesetzt werden dürfen. Es muss also hinsichtlich des ersten Wirkstoffes eine Förderung der Freisetzung jedenfalls im Darm und hinsichtlich des zweiten Wirkstoffes eine Retardierung erzielt werden.

Beispielsweise beschreibt WO 2006/099865 die Freisetzung von zwei Wirkstoffen. Jedoch werden bereits nach wenigen Stunden ein Großteil der Wirkstoffe freigesetzt. Wünschenswert wäre eine verlängerte Freisetzung und gleichzeitig verbesserte optimale Abstimmung der Freisetzung von Wirkstoffen mit unterschiedlichem Lösungsverhalten.

Es ist eine erfindungsgemäße Aufgabe, Arzneiformen bereit zu stellen, die eine verlängerte Freisetzung eines bei hohen pH-Werten schlecht löslichen Wirkstoffes ermöglichen. Bestimmte Ausführungsformen lösen außerdem die Aufgabe, einen bei hohen pH-Werten schlecht löslichen Wirkstoff und einen Wirkstoff mit höherer Löslichkeit gleichermaßen verlängert frei zu setzen. Mit "frei setzen" ist in diesem Zusammenhang gemeint, dass der Wirkstoff am Ort der Resorption, also im Dünndarm, verfügbar gemacht wird. Die erfindungsgemäßen Arzneiformen haben eine lange Verweildauer im Magen, so dass sie hinreichend lang im Magen sind, dass sich ein signifikanter Anteil des ersten Wirkstoffes lösen kann.

Die oben skizzierten Aufgaben werden durch die Gegenstände der Patentansprüche gelöst. Die Arzneiform dieser Erfindung enthält wenigstens einen ersten Wirkstoff und wenigstens einen Emulgator, beide wie in Anspruch 1 definiert, wobei das Massenverhältnis von Wirkstoff zu Emulgator wenigstens 1 zu 30 und höchstens 1 zu 1 beträgt.

Die Arzneiform kann entweder nur ein Kompartiment mit dem ersten Wirkstoff aufweisen oder mehrere Kompartimente aufweisen, die weitere Wirkstoffe enthalten können. Erfindungsgemäß weist die Arzneiform wenigstens ein erstes Kompartiment und ein zweites Kompartiment auf. Erfindungsgemäß weist das erste Kompartiment wenigstens den ersten Wirkstoff auf. In ganz besonders bevorzugten Ausführungsformen besteht die erfindungsgemäße Arzneiform aus dem ersten wirkstoffhaltigen Kompartiment und dem zweiten wirkstoffhaltigen Kompartiment.

Die Arzneiform dieser Erfindung ist eine Schichttablette. Die erfindungsgemäße Arzneiform ist vorzugsweise so gestaltet, dass sie eine Masse von 1750 mg, bevorzugt 1500 mg, weiter bevorzugt 1400 mg, noch weiter bevorzugt 1200 mg und besonders bevorzugt 1000 mg nicht übersteigt. Ihre Masse beträgt formulierungsbedingt bevorzugt wenigstens 700 mg, weiter bevorzugt wenigstens 800 mg. Dabei beträgt die Masse des zweiten Kompartiments, sofern vorhanden, vorzugsweise wenigstens 150 mg, weiter bevorzugt wenigstens 200 mg und insbesondere höchstens 500 mg, bevorzugt höchstens 400 mg und weiter bevorzugt höchstens 350 mg.

Die erfindungsgemäße Arzneiform ist eine Schichttablette, die wenigstens zwei Schichten aufweist, wobei die erste Schicht das oben beschriebene erste Kompartiment ist; und wobei die zweite Schicht das oben beschriebene zweite Kompartiment ist. Ferner kann eine solche Schichttablette auch weitere Schichten enthalten. Ganz besonders bevorzugt ist die erfindungsgemäße Arzneiform eine Schichttablette, die aus zwei Schichten besteht, wobei eine Schicht das erste Kompartiment ist und die andere Schicht das zweite Kompartiment ist.

Bei der Schichttablette kann es sich auch um eine Mantel-Kern-Tablette handeln. Dabei ist eine Schicht im Kern angeordnet und eine weitere Schicht bildet den Mantel. Mit anderen Worten, die Schichttablette kann so ausgestaltet sein, dass eine Schicht die andere Schicht komplett umgibt. Bevorzugt ist aber eine Ausführungsform, bei der die Schichten zwar aneinander grenzen, wobei die eine Schicht die andere allerdings nicht vollständig umgibt. Die eine Schicht umgibt die andere Schicht "nicht vollständig", wenn vorzugsweise nicht mehr als 70%, weiter bevorzugt maximal 62% und ganz besonders bevorzugt maximal 52% der gesamten Außenfläche der einen Schicht von der anderen Schicht bedeckt sind.

In besonders bevorzugten Ausführungsformen handelt es sich bei der hier beschriebenen Arzneiform um eine Magenverweilform. Das bedeutet, dass die Arzneiform sich im Magen nicht so stark auflöst, dass sie den Magen schnell durch den Pylorussphinkter verlassen kann. Vorzugsweise verweilt die erfindungsgemäße Arzneiform für wenigstens 2 Stunden, weiter bevorzugt wenigstens 3 Stunden im Magen des Patienten, bevor sie in den Darm gelangt. Dies wird erfindungsgemäß dadurch erzielt, dass die Arzneiform so ausgestaltet ist, dass sie sich nur sehr langsam auflöst. Dadurch behält sie über einen längeren Zeitraum eine Größe, die es ihr nicht erlaubt, den Magen zu verlassen.

Die Arzneiform hat vorzugsweise an wenigstens einer Stelle einen Durchmesser von mehr als 2 mm, insbesondere mehr als 4 mm und besonders bevorzugt wenigstens 5 mm. Dadurch wird sichergestellt, dass die Arzneiform den Magen nicht sofort durch den Pylorussphinkter verlassen kann, sondern noch eine Weile im Magen bleibt.

Der Magen entleert sich nach der Nahrungsaufnahme nicht sofort, sondern gibt den Speisebrei (Chymus) nur nach und nach an den Dünndarm ab. Diese physiologische Gegebenheit kann sich die erfindungsgemäße Arzneiform zu Nutze machen. Wie oben beschrieben ist der erste Wirkstoff vorzugsweise ein Wirkstoff, der sich bei einem niedrigen pH-Wert, also im sauren Milieu besser als im alkalischen Milieu löst.

Im Magen herrscht typischerweise ein niedriger pH-Wert. Nach dem Essen steigt der pH allerdings auf höhere Werte an, z.B. um pH 5. Erst nach und nach sinkt der pH wieder auf niedrigere Werte. Das heißt, dass sich der erste Wirkstoff nach der Nahrungsaufnahme zunächst schlecht lösen würde, weil der pH-Wert zu hoch ist. Die erfindungsgemäße Arzneiform ermöglicht nun aber die Auflösung eines Teils des ersten Wirkstoffes durch die hierin beschriebenen Maßnahmen.

Durch die Magenbewegung mischt sich der freigesetzte erste Wirkstoff mit dem Chymus und wird dann nach und nach an den Dünndarm abgegeben. Der Wirkstoff wird dann im proximalen Dünndarm, also kurz nach dem Verlassen des Magens, resorbiert. Der Magen dient somit als Retardierungsmittel für den ersten Wirkstoff. Gleichzeitig löst sich, sofern vorhanden, auch der zweite Wirkstoff, der vorzugsweise eine höhere Löslichkeit aufweist als der erste Wirkstoff. Eine höhere Löslichkeit hat der zweite Wirkstoff vorzugsweise dann, wenn seine Löslichkeit um wenigstens eine Zehnerpotenz höher ist als die Löslichkeit des ersten Wirkstoffs.

Sobald der pH-Wert im Magen einen niedrigeren Wert, z.B. etwa pH 3, erreicht hat, löst sich der erste Wirkstoff besser. Damit nun nicht der gesamte Restgehalt des ersten Wirkstoffes auf einmal freigesetzt wird, ist der Emulgator vorzugsweise so gewählt, dass er die Freisetzung des ersten Wirkstoffes verlangsamen kann. Optional unterstützen wenigstens ein Tensid, wenigstens ein Triglycerid und/oder wenigstens ein Verzögerungsmittel dabei.

Im zweiten Kompartiment wird kein Emulgator benötigt, dort ist keiner vorhanden. Stattdessen befindet sich dort vorzugsweise wenigstens ein Retardierungsmittel, das geeignet ist, die Freisetzung des zweiten Wirkstoffes zu verlangsamen, damit hier eine verlängerte Freisetzung erzielt werden kann.

Da die Arzneiform sich nur sehr langsam auflöst, bleibt sie vorzugsweise im Magen bis sich dieser weitgehend entleert hat. Der Speisebrei ist dann zu einem großen Anteil über den Pylorussphinkter in den Dünndarm gelangt. Der Magen leert sich in einem solchen Moment mittels sogenannter Housekeeper-Kontraktionen. Dadurch gelangen auch Körper, die normalerweise nicht über den Pylorussphinkter in den Dünndarm übergehen, dorthin.

Die Arzneiform wird dann weiter im Dünndarm die Wirkstoffe freisetzen. So wird eine nahezu lineare Freisetzung der beiden Wirkstoffe möglich.

Der erste Wirkstoff ist ein Wirkstoff, der in basischem Milieu schlecht löslich ist. Das bedeutet insbesondere, dass der erste Wirkstoff in Wasser mit einem pH-Wert von 7 eine Löslichkeit von weniger als 0,5 Mol/l, insbesondere weniger als 0,1 Mol/l aufweist sowie vorzugsweise weniger als 0,01 Mol/l. Sofern nichts anderes angegeben ist, wird die Löslichkeit bei Normbedingungen (DIN 1343) bestimmt, dies gilt auch für alle anderen Parameter in dieser Beschreibung.

Dies trifft regelmäßig auf Cinnarizin und dessen Salze zu.

Erfindungsgemäß bedeutet "gut löslich" oder "gute Löslichkeit", dass der so bezeichnete Stoff sich in dem entsprechenden Milieu bzw. Lösungsmittel in einer Menge von mehr als 0,5 Mol/l löst. "Schlecht löslich" oder "schlechte Löslichkeit" bedeutet, dass der so bezeichnete Stoff sich in dem entsprechenden Milieu bzw. Lösungsmittel in einer Menge von höchstens 0,5 Mol/l, insbesondere höchstens 0,1 Mol/l und vorzugsweise höchstens 0,01 Mol/l löst.

Wie zuvor erwähnt, profitieren besonders lipophile Wirkstoffe von der Wirkstoffmatrix dieser Erfindung. Der Vorteil für solche Wirkstoffe ist auch deswegen besonders hoch, weil üblicherweise die Resorption lipophiler Wirkstoffe sehr gut funktioniert, sobald diese Substanzen gelöst sind. Mit anderen Worten ist die Auflösung dieser Wirkstoffe aus einer Arzneiform der geschwindigkeitsbestimmende Schritt bei der Aufnahme des Wirkstoffes in den Körper.

Trotzdem gibt es auch Wirkstoffe, deren Lipophilie so ausgeprägt ist, dass die hier vorgeschlagene Wirkstoffmatrix nicht ausreicht, um eine ausreichende Bioverfügbarkeit sicher zu stellen. Daher weisen die Wirkstoffe, die erfindungsgemäß eingesetzt werden, n-Octanol-Wasser-Verteilungskoeffizienten (logPow) bei 20 °C von vorzugsweise höchstens 100, weiter bevorzugt höchstens 50, insbesondere höchstens 30, besonders bevorzugt höchstens 15 und insbesondere höchstens 7 auf.

Um die Löslichkeit des ersten Wirkstoffes nach Applikation zu verbessern, ist es erfindungsgemäß bevorzugt, den ersten Wirkstoff in kleiner Partikelgröße zu verwenden. Daher weist der erste Wirkstoff in der Arzneiform dieser Erfindung vorzugsweise mittlere Partikelgrößen von höchstens 1000 nm, weiter bevorzugt höchstens 600 nm und besonders bevorzugt höchstens 300 nm auf. Die mittlere Partikelgröße wird erfindungsgemäß bevorzugt mit der Analysemethode der dynamischen Lichtstreuung ermittelt.

Die kleine Partikelgröße ist insbesondere dann von Bedeutung, wenn sich der Wirkstoff im Emulgator nicht oder kaum löst. Dies trifft meist zu, wenn der Emulgator einen besonders großen hydrophilen Anteil hat, wie etwa viele Poloxamere, und/oder der Wirkstoff besonders lipophil ist. Es ist erfindungsgemäß bevorzugt, dass sich der Wirkstoff im Emulgator nahezu vollständig, vorzugsweise vollständig, löst. Dadurch wird eine verbesserte Freisetzung erreicht. Erzielt werden kann dieser Effekt einfach dadurch, dass der lipophile Anteil des Emulgators an die Lipophilie des Wirkstoffes angepasst wird. Wie das erreicht werden kann, ergibt sich aus den weiter unten folgenden Ausführungen zum Emulgator und dessen Molekülstruktur.

Besonders gut geeignet ist die erfindungsgemäße Arzneiform zur Verabreichung von Wirkstoffen, die wenigstens eine Aminogruppe aufweisen. Vorteilhaft ist es, wenn diese Wirkstoffe keine Hydroxylgruppen und/oder Carboxylgruppen aufweisen. Jedenfalls profitieren solche Wirkstoffe besonders vom Design der erfindungsgemäßen Arzneiform. Der erste Wirkstoff weist also vorzugweise keine Hydroxylgruppe und/oder Carboxylgruppe auf.

Erfindungsgemäß ist der erste Wirkstoff Cinnarizin oder ein CinnarizinsalzDer erste Wirkstoff wird vorzugsweise in einer Menge von wenigstens 10 mg und weiter bevorzugt wenigstens 20 mg oder besonders bevorzugt wenigstens 40 mg in der Arzneiform eingesetzt. Die Mengen an erstem Wirkstoff soll vorzugsweise einen Wert von 200 mg, weiter bevorzugt 100 mg und besonders bevorzugt 80 mg nicht übersteigen.

Der Emulgator unterstützt die Freisetzung des ersten Wirkstoffes. Dies ist besonders wichtig, damit sich wenigstens ein Teil des ersten Wirkstoffs möglichst zügig und gleichmäßig bereits im Magen auflöst. Wie oben beschrieben, sinkt der pH-Wert des Magens nach Einnahme einer Mahlzeit nur langsam wieder. Es ist aber erforderlich, dass sich der erste Wirkstoff bereits jetzt im Magen löst, damit er mit dem Speisebrei vermischt wird. Das Vermischen erfolgt durch die Magenbewegung. Die Verwendung eines erfindungsgemäßen Emulgators in den erfindungsgemäßen Arzneiformen ermöglicht insbesondere eine Löslichkeitsverbesserung des ersten Wirkstoffs bei höheren pH-Werten. Der erfindungsgemäße Emulgator trägt dazu bei, dass der erste Wirkstoff idealerweise bereits in gelöster Form freigesetzt wird und anschließend resorbiert werden kann.

Damit die Verbesserung der Freisetzung besonders gut ausgeprägt ist, soll der Emulgator vorzugsweise einen HLB-Wert von wenigstens 1 und höchstens 16, insbesondere wenigstens 9 und höchstens 14 aufweisen. Der Emulgator ist vorzugsweise nichtionisch.

Es hat sich gezeigt, dass solche Emulgatoren besonders vorteilhafte Ergebnisse erzielen, die ein bestimmtes Strukturmotiv aufweisen. Dieses Strukturmotiv ist eine Polyethylenglykolkette. Es wurde gefunden, dass Emulgatoren mit diesem Strukturmotiv eine Doppelfunktion besitzen. Einerseits führen sie bei hohen pH-Werten ab etwa pH 4 zu einer Verbesserung der Löslichkeit des ersten Wirkstoffes. Andererseits führen sie bei niedrigen pH-Werten, nämlich dann, wenn der erste Wirkstoff besser löslich ist, zu einer Verzögerung der Freisetzung. Dies ist ein erfindungsgemäß erwünschtes Verhalten, soll doch die Freisetzung des ersten Wirkstoffes möglichst kontinuierlich über einen langen Zeitraum erfolgen.

Die Polyethylenglykolkette stellt den hydrophilen Teil des Emulgators dar und ist mit einem lipophilen Teil verbunden. Der lipophile Teil kann vorteilhafterweise ein Glyceridrest oder eine andere Polyalkylenoxidkette sein.

Als Glyceridreste kommen Mono- und Diglyceridreste in Frage, wobei Diglyceridreste bevorzugt sind. Monoglyceridreste sind unter Umständen, je nach Kettenlänge der beteiligten Fettsäurereste nicht lipophil genug.

Als Polyalkylenoxidketten kommen insbesondere Polyalkylenoxide in Frage, die ununterbrochene Kohlenstoffketten von wenigstens 3 Kohlenstoffatomen aufweisen. Ein bevorzugtes Beispiel für eine Polyalkylenoxidkette im Emulgator dieser Erfindung ist Polypropylenoxid.

Diese Emulgatoren können mit dem Fachmann bekannten Verfahren erhalten werden. Erfindungsgemäße Polyethylenglykolglyceride werden durch Reaktion von den entsprechenden Glyceriden mit Polyethylenglykol hergestellt. Kommerziell erhältliche Polyethylenglykolglyceride sind beispielsweise Gelucire® 43/01 und Gelucire® 50/13.

Die hier beschriebenen Emulgatoren lassen sich durch Reaktion, insbesondere Alkoholyse, von Mono-, Di- und/oder Triglyceriden mit Polyalkylenoxiden herstellen. Diese Edukte sind kommerziell erhältlich. Die erfindungsgemäßen Emulgatoren sind vorzugsweise Mischungen der Reaktionsprodukte der genannten Substanzen.

In bevorzugten Ausführungsformen ist der Emulgator ausgewählt aus Gelucire® 50/13, Gelucire® 43/01, Poloxamer 407 und Mischungen daraus.

Eine gute Lösungsvermittlung wird erzielt, wenn das Verhältnis der Massen von Wirkstoff zu Emulgator wenigstens 1 zu 30, weiter bevorzugt wenigstens 1 zu 20 und besonders bevorzugt wenigstens 1 zu 10 und noch weiter bevorzugt wenigstens 1 zu 8 beträgt. Das genannte Verhältnis beträgt höchstens 1 zu 1, bevorzugt weniger als 1 zu 1, weiter bevorzugt höchstens 1 zu 2, noch weiter bevorzugt höchstens 1 zu 3 und noch weiter bevorzugt höchstens 1 zu 4 und besonders bevorzugt höchstens 1 zu 6. Grundsätzlich gilt, dass eine größere Menge an Emulgator eine bessere Lösungsvermittlung ermöglicht. Es muss aber berücksichtigt werden, dass eine Arzneiform bestimmte Höchstvolumina nicht überschreiten sollte, damit die Einnahme nicht unnötig unangenehm ist. Außerdem kann eine zu große Menge an Emulgator auch dazu führen, dass die Freisetzung des ersten Wirkstoffes behindert wird. Als besonders geeignet hat sich ein Massenverhältnis von Wirkstoff zu Emulgator von wenigstens 1 zu 10 und höchstens 1 zu 2 erwiesen, idealerweise ein Massenverhältnis von wenigstens 1 zu 8 und höchstens 1 zu 2. Bei Einhaltung dieser Massenverhältnisse zeigte sich eine besonders vorteilhafte Freisetzungsbeschleunigung bei besonders günstiger Größe der Arzneiform.

Erfindungsgemäß umfasst das erste Kompartiment den Emulgator. Die Arzneiform oder das erste Kompartiment enthält den Emulgator vorzugsweise in einem Anteil von wenigstens 7 Gew.-%, bevorzugt von wenigstens 10 Gew.-%, weiter bevorzugt von wenigstens 15 Gew.-% und besonders bevorzugt von wenigstens 20 Gew.-%. Der Anteil des Emulgators soll vorzugsweise einen Anteil von 50 Gew.-%, weiter bevorzugt 40 Gew.-% und besonders bevorzugt 32 Gew.-% nicht überschreiten. Beim Versuch, das optimale Verhältnis von Lösungsvermittlung und Volumen der Arzneiform zu ermitteln, haben sich diese Mengen als vorteilhaft erwiesen.

In besonders bevorzugten Ausführungsformen wird zusätzlich zu den oben genannten Substanzen mindestens ein Phospholipid eingesetzt. Das Phospholipid ist vorzugsweise ausgewählt aus Phosphatidylcholinen (Lecithine), Phosphatidylethanolaminen (Kephaline), Phosphatidylserinen, Sphingomyelinen und Mischungen davon. Besonders bevorzugt sind Phosphatidylcholine (Lecithine). Es wurde überraschend gefunden, das Phospholipide ebenfalls die oben beschriebenen vorteilhaften Wirkungen hervorrufen, wie die oben genannten Emulgatoren.

Der weitere Emulgator kann also ein Phospholipid sein. Das Phospholipid verstärkt dabei die vorteilhaften Wirkungen der oben genannten Substanzen.

In bevorzugten Ausführungsformen enthält die Arzneiform ferner wenigstens ein Tensid. Das Tensid dient dazu, die durch den Emulgator vermittelte Lösungsvermittlung zu verstärken. Wird nämlich eine Mischung von Emulgator und Tensid eingesetzt, so wirken diese beiden Stoffe synergistisch. Dadurch kann die Gesamtmasse von Emulgator und Tensid im Verhältnis zum Wirkstoff verringert werden. Wie oben erwähnt kann es zur optimalen Lösungsvermittlung notwendig sein, eine sehr große Menge an Emulgator im Verhältnis zum Wirkstoff zu wählen. Diese große Menge kann durch den Einsatz eines Tensids verringert werden.

Bevorzugte Tenside sind ionische Tenside, insbesondere anionische Tenside. Metallseifen haben sich als besonders vorteilhaft erwiesen. Unter Metallseifen werden vorzugsweise Substanzen verstanden, die als anionische Komponente eine organische Säure und als kationische Komponente ein Metallkation umfassen. Das Metallkation ist vorzugsweise ausgewählt aus Alkali- und Erdalkalimetallionen. Besonders bevorzugt sind Alkalimetallionen und insbesondere Natrium und Kalium. Unter den Erdalkalimetallionen sind Magnesium und Calcium besonders bevorzugt.

Bevorzugte organische Säuren, die als anionische Komponente in den Tensiden vorkommen können, sind Säuren mit wenigstens 6 Kohlenstoffatomen. Organische Säuren mit kürzeren Kohlenstoffkettenlängen haben sich als weniger wirksam erwiesen. Die organischen Säuren sollen allerdings vorzugsweise Kettenlängen von 22 Kohlenstoffatomen nicht überschreiten. Bei längeren Kohlenstoffketten ist der synergistische Effekt im Zusammenspiel mit dem Emulgator nicht besonders stark ausgeprägt. Besonders bevorzugt weisen die organischen Säuren in den Tensiden Kettenlängen von wenigstens 10 und höchstens 18, weiter bevorzugt wenigstens 12 und höchstens 16 Kohlenstoffatomen auf.

Die organischen Säuren können verzweigte oder unverzweigte Kohlenstoffketten aufweisen, bevorzugt sind die Kohlenstoffketten unverzweigt. Ein besonders bevorzugtes Tensid ist Natriummyristat.

Die erfindungsgemäßen Tenside haben ferner die Aufgabe, die Magen-Darm-Passage des Speisebreis und damit die Resorption des ersten Wirkstoffes zu verlangsamen. Es wurde interessanterweise gefunden, dass dies besonders durch die hier beschriebenen Metallseifen erzielt werden kann und dass diese eine direkte Wirkung auf die Steuerung der Gastrointestinalpassage haben können mit der Folge, dass insgesamt mehr an erstem Wirkstoff resorbiert werden kann.

Der Anteil des Tensids an der erfindungsgemäßen Arzneiform soll vorzugsweise wenigstens 2 Gew.-%, bevorzugt wenigstens 7 Gew.-%, weiter bevorzugt wenigstens 12 Gew.-% und besonders bevorzugt wenigstens 17 Gew.-% betragen. Es hat sich gezeigt, dass die Effekte des Tensids so besonders ausgeprägt zutage treten. Eine Menge von vorzugsweise 35 Gew.-%, weiter bevorzugt 25 Gew.-% und besonders bevorzugt 20 Gew.-% sollte aber nicht überschritten werden. Dadurch würde die Freisetzung des ersten Wirkstoffes zu stark behindert. Die absolute Masse des Tensids in der Arzneiform soll einen Wert von 300 mg, weiter bevorzugt 250 mg und besonders bevorzugt 200 mg nicht überschreiten. Die Mindestmenge des Tensids soll vorzugsweise einen Wert von 10 mg, weiter bevorzugt 50 mg und besonders bevorzugt 100 mg nicht unterschreiten.

Der Gehalt an Tensid sollte im Masseverhältnis zum ersten Wirkstoff einen Anteil von 1 zu 1, bevorzugt 1,5 zu 1 und besonders bevorzugt 2 zu 1 nicht unterschreiten. Bevorzugt soll dieses Verhältnis einen Wert von 5 zu 1, weiter bevorzugt 4 zu 1 und insbesondere 3 zu 1 nicht überschreiten. Werden diese Werte berücksichtigt, kann die oben skizzierte vorteilhafte Wirkung optimal genutzt werden.

In einer bevorzugten Ausführungsform weist die Arzneiform dieser Erfindung wenigstens ein Triglycerid auf. Das Triglycerid dient dazu, die Magen-Darm-Passage der Arzneiform zu verlangsamen. Das Triglycerid kann auch in Kombination mit dem Tensid eingesetzt werden, um die Freisetzung des ersten Wirkstoffes weiter zu verlangsamen.

Das Triglycerid umfasst vorzugsweise einen Fettsäurerest mit wenigstens 10 Kohlenstoffatomen, weiter bevorzugt wenigstens 12 Kohlenstoffatomen und besonders bevorzugt wenigstens 16 Kohlenstoffatomen. Diese Mindestkettenlänge ist wünschenswert, weil damit die Verlängerung der Verweildauer der Arzneiform im Magen besonders ausgeprägt ist und die Freisetzung des ersten Wirkstoffes so besonders effizient unterstützt werden kann. Allerdings sollte die Kettenlänge vorzugsweise einen Wert von 22 Kohlenstoffatomen, weiter bevorzugt 20 Kohlenstoffatomen nicht überschreiten. In besonders bevorzugten Ausführungsformen erfüllen wenigstens zwei und ganz besonders bevorzugt alle Fettsäurereste in dem Triglycerid diese Voraussetzungen.

Das Triglycerid kann vorzugsweise in einem Anteil von mehr als 5 Gew.-%, bevorzugt wenigstens 7 Gew.-%, weiter bevorzugt wenigstens 10 Gew.-%, noch weiter bevorzugt wenigstens 15 Gew.-% und besonders bevorzugt wenigstens 20 Gew.-% in der erfindungsgemäßen Arzneiform zum Einsatz kommen. Ein Höchstanteil von vorzugsweise 40 Gew.-%, weiter bevorzugt 30 Gew.-% und besonders bevorzugt 25 Gew.-% sollte aber nicht überschritten werden, weil andernfalls die Freisetzung des ersten Wirkstoffes zu stark eingeschränkt würde.

Die absolute Masse des Triglycerids in der Arzneiform soll einen Wert von 500 mg, weiter bevorzugt 400 mg und besonders bevorzugt 300 mg nicht überschreiten. Die Mindestmenge des Triglycerids soll vorzugsweise einen Wert von 50 mg, weiter bevorzugt 100 mg und besonders bevorzugt 150 mg nicht unterschreiten.

Der Gehalt an Triglycerid im Massenverhältnis zum ersten Wirkstoff beträgt bevorzugt mehr als 1 zu 1. Der Gehalt an Triglycerid sollte im Masseverhältnis zum ersten Wirkstoff weiter bevorzugt einen Anteil von 2 zu 1, noch weiter bevorzugt 3 zu 1 und besonders bevorzugt 3,5 zu 1 nicht unterschreiten. Bevorzugt soll dieses Verhältnis einen Wert von 20 zu 1, weiter bevorzugt 10 zu 1, noch weiter bevorzugt 7 zu 1 und insbesondere 6 zu 1 nicht überschreiten. Werden diese Werte berücksichtigt, kann die oben skizzierte vorteilhafte Wirkung optimal genutzt werden.

Das erste Kompartiment kann ein Presshilfsmittel umfassen. Das Presshilfsmittel ist bevorzugt ausgewählt aus Lactose und Maisstärke. Das Presshilfsmittel ist weiter bevorzugt Maisstärke. In einer anderen Ausführungsform wird Lactose als Presshilfsmittel eingesetzt. Das Presshilfsmittel dient unter anderem dazu, den Schmelzpunkt einer Mischung von Wirkstoff und Emulgator während der Herstellung anzuheben, so dass eine weitere Bearbeitung dieser Mischung möglich ist.

Der Anteil dieses Presshilfsmittels an der Arzneiform, oder wenn die Arzneiform mehrere Kompartimente hat, an dem ersten Kompartiment soll vorzugsweise wenigstens 10 Gew.-%, weiter bevorzugt wenigstens 20 Gew.-% und besonders bevorzugt wenigstens 30 Gew.-% betragen. Wird eine zu geringe Menge des Presshilfsmittels eingesetzt, wird der gewünschte Effekt nicht erreicht. Eine zu große Menge an Presshilfsmittel vergrößert die Arzneiform und ist daher unerwünscht. Daher soll der Anteil an Presshilfsmittel an der Arzneiform, oder wenn die Arzneiform mehrere Kompartimente hat, an dem ersten Kompartiment, einen Wert von 65 Gew.-%, bevorzugt 60 Gew.-%, weiter bevorzugt 50 Gew.-% und besonders bevorzugt 40 Gew.-% nicht übersteigen.

Die Arzneiform dieser Erfindung kann auch ein Verzögerungsmittel enthalten. Das Verzögerungsmittel ist vorzugsweise ausgewählt aus polymeren organischen Substanzen, insbesondere solchen, die quellfähig sind. Bevorzugte Verzögerungsmittel sind Acrylatpolymere, Methacrylatpolymere und deren Copolymere sowie Mischungen daraus. Andere bevorzugte Verzögerungsmittel sind Cellulosen, insbesondere hochviskose Cellulosen. Das Verzögerungsmittel der Arzneiform dieser Erfindung ist vorzugsweise ein Polymer, insbesondere ein Polysaccharid. Bevorzugt sind Cellulose, Cellulosederivate, Alginate und Mischungen daraus sowie deren Hydrate, es kommen aber auch Polyacrylate, Polymethacrylate und deren Copolymere und Mischungen in Frage. Besonders bevorzugte Cellulosederivate sind Methylcellulose und Hydroxypropylmethylcellulose.

Bevorzugt wird ein Verzögerungsmittel verwendet, dass in einer wässrigen Lösung in einem Anteil von 2 Gew.-% bei einer Temperatur von 20°C und einem Druck von 101,325 kPa eine Viskosität von wenigstens 1500 mPas aufweist. Die Viskosität wird mit einem Kugelfallviskosimeter (DIN 53015) gemessen. Je nach gewünschter Verlängerung der Freisetzung des ersten Wirkstoffes kann es erwünscht sein, auch höhere Viskositäten zu wählen. Je höher die Viskosität des Verzögerungsmittels, desto stärker wird die Verlängerung der Freisetzung ausfallen. In besonders bevorzugten Ausführungsformen beträgt die Viskosität des Verzögerungsmittels sogar wenigstens 2500 mPas und besonders bevorzugt wenigstens 3500 mPas. Ist die Viskosität allerdings zu hoch, wird der erste Wirkstoff zu langsam freigesetzt. Dies würde dazu führen, dass der erste Wirkstoff während seiner Passage durch den Magen-Darm-Trakt unter Umständen nicht vollständig freigesetzt wird. Daher soll die Viskosität auf höchstens 200 000 mPas, weiter bevorzugt höchstens 120 000 mPas beschränkt sein.

Der Anteil dieses Verzögerungsmittels an der Arzneiform, oder wenn die Arzneiform mehrere Kompartimente hat, an dem ersten Kompartiment, soll vorzugsweise wenigstens 10 Gew.-%, weiter bevorzugt wenigstens 20 Gew.-% und besonders bevorzugt wenigstens 30 Gew.-% betragen. Wird eine zu geringe Menge des Verzögerungsmittels eingesetzt, wird der gewünschte Effekt nicht erreicht. Bei zu großen Mengen wird der erste Wirkstoff nicht schnell genug freigesetzt. Daher soll der Anteil an Verzögerungsmittel an der Arzneiform, oder wenn die Arzneiform mehrere Kompartimente hat, an dem ersten Kompartiment, einen Wert von 60 Gew.-%, weiter bevorzugt 50 Gew.-% und besonders bevorzugt 40 Gew.-% nicht übersteigen. Im Masseverhältnis zum ersten Wirkstoff soll der Gehalt an Verzögerungsmittel vorzugsweise wenigstens 3 zu 1, weiter bevorzugt wenigstens 5 zu 1 und besonders bevorzugt wenigstens 7 zu 1 betragen. Allerdings sollte dieses Verhältnis einen Wert von 20 zu 1, weiter bevorzugt 15 zu 1 und besonders bevorzugt 10 zu 1 nicht überschreiten.

Ferner kann dem ersten und/oder dem zweiten Kompartiment ein Fettalkohol zugesetzt werden. Der Fettalkohol ist dabei vorzugsweise ein Alkohol mit einer Kettenlänge von wenigstens 10 Kohlenstoffatomen, weiter bevorzugt wenigstens 12 Kohlenstoffatomen und besonders bevorzugt wenigstens 14 Kohlenstoffatomen. Die Kettenlänge soll allerdings einen Wert von vorzugsweise 20 Kohlenstoffatomen, weiter bevorzugt 18 Kohlenstoffatomen und insbesondere 16 Kohlenstoffatomen nicht überschreiten. Es hat sich gezeigt, dass die Zugabe eines Fettalkohols geeignet ist, die Freisetzung der Wirkstoffe, insbesondere des zweiten Wirkstoffes, zu verzögern.

Der Gewichtsanteil des Fettalkohols an der Arzneiform bzw. dem ersten Kompartiment (sofern die Arzneiform mehrere Kompartimente hat) beträgt vorzugsweise wenigstens 5 Gew.-%, weiter bevorzugt wenigstens 10 Gew.-% und besonders bevorzugt wenigstens 18 Gew.-%. Bei einem zu geringen Anteil des Fettalkohols an der Arzneiform bzw. dem ersten Kompartiment (sofern die Arzneiform mehrere Kompartimente hat) kann die erfindungsgemäße Wirkung beeinträchtigt sein. Bei einem zu hohen Gehalt kann die strukturelle Identität der Arzneiform beeinträchtigt werden. Daher beträgt der Gehalt des Fettalkohols an der Arzneiform bzw. dem ersten Kompartiment (sofern die Arzneiform mehrere Kompartimente hat) vorzugsweise höchstens 40 Gew.-%, weiter bevorzugt höchstens 35 Gew.-% und besonders bevorzugt höchstens 30 Gew.-%.

Die Arzneiform dieser Erfindung kann weitere pharmazeutische Hilfsstoffe umfassen. Diese Hilfsstoffe können vorzugsweise ausgewählt sein aus Schmiermitteln, Gleitmitteln, Bindemitteln, Sprengmitteln, Antioxidantien, Komplexbildnern, Überzugsmitteln, Fließmitteln, Konservierungsmitteln, Füllstoffen, Weichmachern, Pigmenten und Mischungen aus solchen Substanzen.

Ein bevorzugter Hilfsstoff in der Arzneiform ist ein pharmazeutisch akzeptabler Trägerstoff. Bevorzugte Trägerstoffe sind Polysaccharide, insbesondere Cellulose und/oder Lactose. Der Trägerstoff verleiht der Arzneiform hinreichende Stabilität. Schließlich enthält die Arzneiform einen großen Anteil an Emulgator. Der Emulgator trägt nur sehr eingeschränkt zur strukturellen Integrität der Arzneiform bei. Vorzugsweise enthält die Arzneiform den Trägerstoff in einem Gewichtsanteil von wenigstens 10 Gew.-%, weiter bevorzugt wenigstens 20 Gew.-% und besonders bevorzugt wenigstens 30 Gew.-%.

Zusammenfassend kann die Arzneiform, bzw. das erste Kompartiment, wenn die Arzneiform mehrere Kompartimente hat, unter anderem enthalten:
a. einen ersten Wirkstoff,
b. einen Emulgator zur Verbesserung der Löslichkeit des ersten Wirkstoffes bei hohen pH-Werten und zur Verlangsamung der Auflösung des ersten Wirkstoffes bei niedrigen pH-Werten;
c. optional ein Tensid zur Verbesserung der Löslichkeit des ersten Wirkstoffes und zur Verlangsamung des Transports des ersten Wirkstoffes vom Magen in den Darm;
d. optional ein Triglycerid ebenfalls zur Verbesserung der Löslichkeit des ersten Wirkstoffes und zur Verlangsamung des Transports des ersten Wirkstoffes vom Magen in den Darm;
e. optional ein Verzögerungsmittel zur Verlangsamung der Auflösung des ersten Wirkstoffes bei niedrigen pH-Werten;
f. optional ein Presshilfsmittel zur Unterstützung bei der Verarbeitung einer Mischung des ersten Wirkstoffes und des Emulgators während der Herstellung;
g. optional einen Fettalkohol zur Verlangsamung der Auflösung des ersten Wirkstoffes bei niedrigen pH-Werten;
h. optional weitere Hilfsstoffe.

Die Arzneiform gemäß der Erfindung weist wenigstens zwei Kompartimente auf, die vorzugsweise unterschiedliche Wirkstoffe enthalten. Dabei weist das erste Kompartiment die oben genannten Stoffe auf, nämlich den ersten Wirkstoff und den Emulgator sowie optional Tensid, Triglycerid, Verzögerungsmittel, Presshilfsmittel, Fettalkohol und optionale weitere Hilfsstoffe. Ganz besonders bevorzugt besteht die Arzneiform aus zwei wirkstoffhaltigen Kompartimenten mit unterschiedlichen Wirkstoffen, wobei das erste Kompartiment die oben genannten Stoffe aufweist. Mit "unterschiedliche Wirkstoffe" ist erfindungsgemäß gemeint, dass sich die Wirkstoffe der Kompartimente unterscheiden, also ein und derselbe Wirkstoff nicht in verschiedenen Kompartimenten enthalten ist.

Das erste Kompartiment weist wenigstens den ersten Wirkstoff auf, das zweite Kompartiment weist wenigstens einen zweiten Wirkstoff auf. Neben dem ersten und zweiten Wirkstoff könnten in der Arzneiform weitere Wirkstoffe enthalten sein. Das erste und wenigstens zweite Kompartiment kann also neben dem ersten bzw. zweiten Wirkstoff weitere Wirkstoffe enthalten. Ganz besonders bevorzugt besteht der Wirkstoffanteil der Arzneiform jedoch aus dem ersten und dem zweiten Wirkstoff.

Der Gewichtsanteil des ersten Kompartiments an der erfindungsgemäßen Arzneiform beträgt vorzugsweise 45 bis 100 Gew.-%, weiter bevorzugt 55 bis 87,5 Gew.-%. Hintergrund ist, dass das erste Kompartiment nicht nur die verlängerte Freisetzung, sondern auch die Lösungsvermittlung des schwer zu formulierenden ersten Wirkstoffes ermöglichen muss.

Ein zweites Kompartiment der Arzneiform weist wenigstens den zweiten Wirkstoff auf. Der zweite Wirkstoff ist vorzugsweise ein Wirkstoff mit höherer Wasserlöslichkeit als der erste Wirkstoff, der sich somit sowohl im Magen als auch im Darm besser als der erste Wirkstoff löst. Der zweite Wirkstoff weist also vorzugsweise eine um wenigstens eine Zehnerpotenz höhere Löslichkeit auf als der erste Wirkstoff, insbesondere bei pH 7.

Das zweite Kompartiment kann insbesondere beinhalten:
a. einen zweiten Wirkstoff,
b. optional ein Retardierungsmittel zur Verlangsamung der Freisetzung des zweiten Wirkstoffes,
c. optional einen langkettigen Alkohol zur Verlangsamung der Freisetzung des zweiten Wirkstoffes,
d. optional ein Tensid, wie es oben beschrieben, ist zur Verlangsamung des Transports des Wirkstoffes vom Magen in den Darm;
e. optionale ein Triglycerid wie oben beschrieben ist zur Verlangsamung des Transports des Wirkstoffes vom Magen in den Darm;
f. optional weitere Hilfsstoffe.

Im Stand der Technik wird keine überzeugende Arzneiform vorgestellt, die es ermöglicht, sowohl einen schlecht löslichen Wirkstoff (hier: erster Wirkstoff) als auch einen zweiten Wirkstoff, insbesondere wenn dieser eine höhere Löslichkeit als der erste Wirkstoff hat, in einer Arzneiform jeweils verzögert frei zu setzen. Schließlich muss die Freisetzung des schlecht löslichen Wirkstoffes unterstützt werden und, im Falle eines besser löslichen zweiten Wirkstoffes, gleichzeitig die Freisetzung des zweiten Wirkstoffes unterdrückt werden. Diese Erfindung bietet eine Lösung für dieses Problem, indem eine erfindungsgemäße Arzneiform bereit gestellt wird, die bevorzugt zwei Kompartimente umfasst, wobei das erste Kompartiment den ersten Wirkstoff und das zweite Kompartiment den zweiten Wirkstoff enthält.

Im Kontext der hier beschriebenen Arzneiform ist es eine Herausforderung, die Freisetzung des zweiten Wirkstoffes zu verzögern. Ein Grund ist, dass zur Lösungsvermittlung des ersten Wirkstoffes eine vergleichsweise große Menge an Emulgator benötigt wird. Dies führt dazu, dass das Volumen der Arzneiform bereits durch die notwendige löslichkeitsverbessernde Maßnahme hinsichtlich des ersten Wirkstoffes fast ausgereizt ist. Die freisetzungsverzögernde Maßnahme hinsichtlich des zweiten Wirkstoffes muss also in minimalem Volumen möglich sein.

Ein einfacher Überzug der ganzen Arzneiform ist also nicht bevorzugt, weil dieser Überzug die Freisetzung beider Wirkstoffe unterdrücken würde. Es ist also erfindungsgemäß, dass die Arzneiform zwei Kompartimente umfasst, wobei das erste Kompartiment wenigstens den ersten Wirkstoff und wenigstens einen Emulgator umfasst. Das zweite Kompartiment umfasst den zweiten Wirkstoff. Damit der zweite Wirkstoff verlängert freigesetzt wird, enthält das zweite Kompartiment vorzugsweise ein Retardierungsmittel.

In einer bevorzugten Ausführungsform dieser Erfindung umfasst die Arzneiform wenigstens zwei Kompartimente in Form von Schichten. Dabei stellt vorzugsweise eine der Schichten das erste Kompartiment und eine andere der Schichten das zweite Kompartiment dar. Die Arzneiform kann auch weitere Schichten, insbesondere eine dritte Schicht, umfassen.

In einer Ausführungsform dieser Erfindung umfasst die Arzneiform dieser Erfindung einen Mantel und einen Kern. Dabei kann der Kern ein Kompartiment dieser Erfindung sein, insbesondere das erste Kompartiment. Der Mantel kann ein anderes Kompartiment sein, insbesondere das zweite Kompartiment. Der Mantel umgibt den Kern dabei vorzugsweise vollständig. Mit anderen Worten, der Kern liegt komplett innerhalb des Mantels.

Grundsätzlich kann jeder Wirkstoff von der Darreichung in der erfindungsgemäßen Arzneiform profitieren, der über einen längeren Zeitraum verlängert verabreicht werden soll. Beispiele für Wirkstoffe und Wirkstoffklassen, welche vorteilhaft als zweiter Wirkstoff im Rahmen der vorliegenden Erfindung eingesetzt werden können, sind:
Arzneimittel zur Behandlung von Schmerzen und zur Schmerztherapie mit peripher wirkenden Analgetika, zentral wirkenden Analgetika und adjuvante Nicht-Analgetika. Es handelt sich hier vorzugsweise um folgende Analgetika und adjuvante Stoffe:
Acetylsalicylsäure, Ibuprofen, Diclofenac, Indomethacin, Naproxen, Piroxicam, Paracetamol, Metamizol, Celecoxib, Parecoxib, Tramadol, Pethidin, Codein, Dihydrocodein, Piritramid, Tilidin, Morphin, Hydromorphon, Oxycodon, Levomethadon, Fentanyl, Sufentanil, Buprenorphin, Pentazocin, Naloxon, Flupirtin, Carbamazepin, Metoprolol, Metoclopramid, Amitryptilin, Doxepin, Clomipramin, Mianserin, Maprotilin, Triptane wie z.B. Naratriptan, Rizatriptan, Sumatriptan, Zolmitriptan, Kalziumantagonisten wie: Flunarizin, Topiramat, Valproinsäure, Phenytoin, Baclofen, sonstige Mittel wie: Botulinum Toxin, Ergotamine, Lisurid, Methysergid, Pizotifen, Oxcarbazepin, Gabapentin und Lamotrigin, Dexamethason, Methylprednisolon, Prednisolon, Triamcinolon, Diazepam, Tetrazepam, Tizanidin, Butylscopolamin und/oder Kombination von Tilidin und Naloxon.

Arzneistoffe zur Behandlung des Nervensystems, allein oder in Kombination, z.B. Anfallsleiden (insbesondere Clonazepam, Diazepam, Lorazepam, Midazolam, Clobazam, Phenytoin, Clomethiazol, Valproinsäure, Phenobarbital, Gabapentin, Lamotrigin, Oxcarbazepin, Pregabalin, Topiramat, Ethosuximid, Levetrazetam, Mesuximid, Primidon, Nitrazepam und/oder Vigabitrin), Parkinson-Syndrom (insbesondere Levodopa, mit Benserazid/Carbidopa, Bromocriptin, Cabergolin, Dihydroergocriptin, Lisurid, Pergolidmesilat, Pramipexol, Ropinirol, Apomorphin, Biperiden, Metixenhydrochlorid, Trihexphenidyl, Entacapon, Amantadin, Budidin, Selegilin und/oder Apomorphin), Schlaganfall (insbesondere Acetylsalicylsäure, Clopidogrel, Dipyridamol, Ticlopidin, Heparin, Phenprocoumon, Warfarin, Protamin, Phytomenadion, Nimodipin, Paracetamol, Tramadol und/oder Buprenorphin), Hirndruck (insbesondere Furosemid und/oder Mannitol), Tremor (insbesondere Propranolol, Clozapin, Alprazolam und/oder Primidon).

Arzneistoffe zur Behandlung von psychiatrischen Erkrankungen wie Angststörungen (insbesondere Alprazolam, Diazepam, Fluoxetin, Paroxetin, Chlorprothixen, Levomepromazin, Thioridazin, Flupentixol und/oder Fluspirilen), Depressionen (insbesondere Imipramin, Amitriptylin, Desipramin, Maprotilin, Minaserin, Citalopram, Fluoxetin, Paroxetin, Trazodon, Moclobemid und/oder Miratazepam), Psychosen und Schizophrenien (insbesondere Sulpirid, Promazin, Melperon, Thioridazin, Chlorprothixen, Perazin, Pimozid, Fluphenazin, Olanzapin und/oder Risperidon), Schlafstörungen (insbesondere Triazolam, Brotizolam, Oxazepam, Flurazepam, Nitrazepam, Temazepam, Zolpidemtratrat, Zopiclon, Promethazin, Chlorprothixen, Pipamperon, Thioridazin und/oder Chloralhydrat) Unruhezustände und Verwirrtheit (insbesondere Alprazolam, Oxazepam, Doxepin, Clomipramin, Imipramin, Thioridazin und/oder Perazin), Demenz vom Alzheimer-Typ (insbesondere Donepezil, Rivastigmin, Tacrin, Memantin, Nimodipin und/oder Selegilin).

Arzneistoffe zur Behandlung von Herz-Kreislauf-Krankheiten, wie koronare Herzkrankheit/Angina Pectoris (insbesondere Acetylsalicylsäure, Clopidrogel, Ticlopidin, Isosorbiddinitrat, Isosorbidmononitrat, Nitroglycerin, Molsidomin, Trapidil, Metoprolol, Bisoprolol, Atenolol, Acebutolol, Carvedilol, Nitrendipin, Nifedipin, Diltiazem, Verapamil, Benazepril, Lisinopril, Ramipril, Fosinopril und/oder Enalapril), Herzinfarkt und Herzinsuffizienz (insbesondere Isosorbidmono- und dinitrat, Clopidogrel, Ticlopidin, Captoprol, Ramipril, Lisinopril, Candesartan, Eprosartan, Irbesatan, Losartan, Chlortalidon, Xipamid, Hydrochlorothiazid, Furosemid, Piretanid, Triameteren, Digitalisglycoside, Carvedilol, Metoprolol und/oder Prazosin), Herzrhythmusstörungen (insbesondere Ajmalin, Chinidin, Disopyramid, Flecainid, Propafenon, Propranolol, Carvedilol, Amiodaron, Verapamil und/oder Diltiazem), Hypertonie (insbesondere Metoprolol, Atenolol, Urapidil, Clonidin, Dihydralazin, Chlortalidon, Hydrochlorothiazid, Furosemid, Felodipin, Israpidin, Lacidipin, Diltiazem, Captopril, Enalapril, Fosinopril, Lisinopril, Ramnipril, Verapamil, Candesartan, Eprosartan, Irbesatan, Losartan, Doxazosin, Bunazosin, Prazosin, Terazosin, Moxonidin, Dihydralazin und/oder Minoxidil).

Arzneistoffe, allein oder in Kombination, zur Behandlung der Atemwege und der Lunge (insbesondere Theophyllin, Methylprednisolon, Flucorton, Dexamethason, Montekulast, Roxithromycin, Erythromycin, Azithromycin, Ciprofloxacin, Clarithromycin, Levofloxacin, Ofloxacin, Doxycyclin, Ampicillin und Sulbactam, Amoxicillin, Cefuroxim, Clindamycin, Cefotiam, Cefuroxim, Ceftazidim, Ceftriaxon, Piperacillin und/oder Moxifloxacin).

Arzneistoffe zur Behandlung des Magen-Darm-Traktes und der Bauchspeicheldrüse (insbesondere Fluconazol, Mesalazin, Sulfasalazin, Budenosid, Azathioprin, Prednison, Metronidazol, Infliximab, Loperamid, Cotrimoxazol, Ciprofloxacin, Metronidazol, Vancomycin, Esomeprazol, Lansoparzol, Pantoprazol, Rabeprazol, Cimetidin, Famotidin, Ranitidin, Nizatidin, Sucralfat, Misoprostol, Metoclopramid, Pirenzepin, Bisacodyl, Domperidon, Sulpirid, Alizaprid, Dimenhydrinat, Cinnarizin, Flunarizin, Levomeprazin, Ondansetron, Betahistin und/oder Aprepitant).

Arzneistoffe zur Infektabwehr mit Antibiotika oder antiviral wirksamen Substanzen (insbesondere Acyclovir, Amantadin, Azithromycin, Bacampicillin, Cefaclor, Cefazolin, Cefixim, Cefprozil, Ceftriaxon, Chloroquin, Ciprofloxacin, Clotrimazol, Dicloxacillin, Doxycycllin, Econazol, Erythromycin, Ethambutol, Fosfomycin, Flucloxacillin, Fluconazol, Fusidinsäure, Gramicidin, Idoxuridin, Indinavir, Interferon, Itraconazol, Isoniazid, Josamycin, Ketoconazol, Lamivudin, Lomefloxacin, Mafenid, Mebendazol, Mesalazin, Mezlozillin, Mupirocin, Miconazol, Naftifin, Nalidixinsäure, Norfloxacin, Ofloxacin, Oxacillin, Oxytetracyclin, Piperacillin, Praziquantel, Primaquim, Proguanil, Ribavirin, Rifabutin, Rimantadin, Roxothromycin, Saquinavir, Spectinomycin, Spriramycin, Stavudin, Sulbactam, Teiucoplanin, Terbinafin, Tetracyclin, Tetroxoprim, Ticarcillin, Tinidazol, Tromantadin, Tolnaftat, Vancomycin, Zidovudin und/oder Zalcitabin).

Arzneistoffe zur Behandlung der erektilen Dysfunktion (insbesondere Sildenafil, Tadalafil, Vardenafil, Theobromin, Koffein und/oder Theophyllin).

In bevorzugten Ausführungsformen umfasst die erfindungsgemäße Arzneiform keine Antibiotika in pharmazeutisch wirksamer Menge, insbesondere keinen Vertreter der Wirkstoffklasse der Tetracycline. Antibiotika beeinträchtigen die Darmflora, in dem sie wichtige Bakterien im Darm abtöten. In der Arzneiform dieser Erfindung würde dieser Effekt zu starken Nebenwirkungen führen, weil die Arzneiform sich teilweise erst sehr spät auflöst.

In bevorzugten Ausführungsformen umfasst die erfindungsgemäße Arzneiform keine Protonenpumpenhemmer. Protonenpumpenhemmer erhöhen den pH-Wert im Magen und stören somit die Auflösung vieler Arzneistoffe aus der ersten wirkstoffhaltigen Einheit, insbesondere der schwach basischen Wirkstoffe.

Der zweite Wirkstoff ist bevorzugt in Form kleiner Partikel in dem zweiten Kompartiment enthalten. Das bedeutet, dass die mittlere Partikelgröße des zweiten Wirkstoffes vorzugsweise 1000 nm nicht übersteigt. Weiter bevorzugt beträgt die mittlere Partikelgröße des zweiten Wirkstoffes nicht mehr als 300 nm. Die Partikelgröße des Wirkstoffes wird mittels Laserdiffraktion bestimmt.

Der zweite Wirkstoff kann ein in Wasser gut löslicher Wirkstoff sein. Der zweite Wirkstoff ist insbesondere ein Wirkstoff, der wenigstens eine Hydroxylgruppe, wenigstens eine Carboxylgruppe und/oder wenigstens eine permanente Ladung aufweist. Ein bevorzugter Wirkstoff ist Dimenhydrinat.

Der zweite Wirkstoff wird vorzugsweise in einer Menge von wenigstens 50 mg und weiter bevorzugt wenigstens 80 mg oder besonders bevorzugt wenigstens 100 mg in der Arzneiform eingesetzt. Die Mengen an zweitem Wirkstoff soll vorzugsweise einen Wert von 400 mg, weiter bevorzugt 300 mg und besonders bevorzugt 200 mg nicht übersteigen.

Das Retardierungsmittel in dem zweiten Kompartiment der Arzneiform dieser Erfindung ist vorzugsweise ein Polymer, insbesondere ein Polysaccharid. Bevorzugt sind Cellulosen, Cellulosederivate, Alginate oder Mischungen daraus, es kommen aber auch Polyacrylate, Polymethacrylate und deren Copolymere und Mischungen in Frage. Besonders bevorzugte Cellulosederivate sind Methylcellulose und Hydroxypropylmethylcellulose. Solche Cellulosederivate ermöglichen eine besonders vorteilhafte Freisetzung des zweiten Wirkstoffs und zeigen kein ungünstiges pH-abhängiges Verhalten.

Bevorzugt wird ein Retardierungsmittel verwendet, dass in einer wässrigen Lösung in einem Anteil von 2 Gew.-% bei einer Temperatur von 20°C und einem Druck von 101,325 kPa eine Viskosität von wenigstens 1500 mPas aufweist. Die Viskosität wird mit einem Kugelfallviskosimeter (DIN 53015) gemessen. Je nach gewünschter Retardierung der Freisetzung des zweiten Wirkstoffes kann es erwünscht sein, auch höhere Viskositäten zu wählen. Je höher die Viskosität des Retardierungsmittels, desto stärker wird die Retardierung der Freisetzung ausfallen. In besonders bevorzugten Ausführungsformen beträgt die Viskosität des Retardierungsmittels sogar wenigstens 2500 mPas und besonders bevorzugt wenigstens 3500 mPas. Ist die Viskosität allerdings zu hoch, wird der zweite Wirkstoff zu langsam freigesetzt. Dies würde dazu führen, dass der zweite Wirkstoff während seiner Passage durch den Magen-Darm-Trakt unter Umständen nicht vollständig freigesetzt wird. Daher soll die Viskosität auf höchstens 200 000 mPas, weiter bevorzugt höchstens 120 000 mPas beschränkt sein.

Der Anteil dieses Retardierungsmittels an dem zweiten Kompartiment soll vorzugsweise wenigstens 10 Gew.-%, weiter bevorzugt wenigstens 20 Gew.-% und besonders bevorzugt wenigstens 30 Gew.-% betragen. Wird eine zu geringe Menge des Retardierungsmittels eingesetzt, wird der erfindungsgemäße Effekt nicht erreicht. Bei zu großen Mengen wird der zweite Wirkstoff nicht schnell genug freigesetzt. Daher soll der Anteil an Retardierungsmittel in dem zweiten Kompartiment einen Wert von 60 Gew.-%, weiter bevorzugt 50 Gew.-% und besonders bevorzugt 40 Gew.-% nicht übersteigen.

Ferner kann dem ersten und/oder dem zweiten Kompartiment ein langkettiger Alkohol zugesetzt werden. "Langkettig" bezeichnet dabei einen Alkohol mit einer Kettenlänge von wenigstens 10 Kohlenstoffatomen, weiter bevorzugt wenigstens 12 Kohlenstoffatomen und besonders bevorzugt wenigstens 14 Kohlenstoffatomen. Die Kettenlänge soll allerdings einen Wert von vorzugsweise 20 Kohlenstoffatomen, weiter bevorzugt 18 und insbesondere 16 Kohlenstoffatomen nicht überschreiten. Es hat sich gezeigt, dass die Zugabe eines langkettigen Alkohols geeignet ist, die Freisetzung der Wirkstoffe, insbesondere des zweiten Wirkstoffes zu verzögern.

Der Gewichtsanteil des langkettigen Alkohols an dem zweiten Kompartiment dieser Erfindung beträgt vorzugsweise wenigstens 5 Gew.-%, weiter bevorzugt wenigstens 10 Gew.-% und besonders bevorzugt wenigstens 18 Gew.-%. Bei einem zu geringen Anteil des langkettigen Alkohols an dem zweiten Kompartiment wird die gewünschte Wirkung nicht erzielt. Bei einem zu hohen Gehalt kann die strukturelle Integrität der Arzneiform beeinträchtigt werden. Daher beträgt der Gehalt des langkettigen Alkohols an dem zweiten Kompartiment vorzugsweise höchstens 40 Gew.-%, weiter bevorzugt höchstens 35 Gew.-% und besonders bevorzugt höchstens 30 Gew.-%.

Das zweite Kompartiment in der erfindungsgemäßen Arzneiform kann ferner weitere Hilfsstoffe aufweisen. Diese Hilfsstoffe können vorzugsweise ausgewählt sein aus Schmiermitteln, Gleitmitteln, Bindemitteln, Sprengmitteln, Antioxidantien, Komplexbildnern, Überzugsmitteln, Fließmitteln, Konservierungsmitteln, Füllstoffen, Weichmachern, Pigmenten und Mischungen aus solchen Substanzen.

Eine besonders bevorzugte Ausführungsform der erfindungsgemäßen Arzneiform umfasst als ersten Wirkstoff Cinnarizin, ganz besonders bevorzugt ist der erste Wirkstoff Cinnarizin und der zweite Wirkstoff Dimenhydrinat. Die Arzneiform ist zur Verwendung in der Therapie von Schwindel jedweder Genese geeignet. Daher ist auch die Verwendung dieser Arzneiform zur Behandlung von Schwindel jedweder Genese erfindungsgemäß.

Ein großer Vorteil der erfindungsgemäßen Arzneiform ist, dass sie sich kostengünstig herstellen lässt.

Das Verfahren zur Herstellung der Arzneiform umfasst vorzugsweise die Schritte:
a. Mischen der Bestandteile der Arzneiform,
b. Granulieren der Mischung und
c. Herstellung einer monolithischen Arzneiform aus dem Granulat.

Das Mischen der Bestandteile der Arzneiform erfolgt in Ausführungsformen, in denen die Arzneiform wenigstens zwei Kompartimente umfasst, vorzugsweise durch Mischen der Bestandteile jedes einzelnen Kompartimentes zum Erhalt von wenigstens zwei Kompartimentmischungen. Das Granulieren der Mischung umfasst in Ausführungsformen, in denen die Arzneiform wenigstens zwei Kompartimente umfasst, vorzugsweise das Granulieren der wenigstens zwei Kompartimentmischungen zum Erhalt von wenigstens zwei Kompartimentgranulaten. Die Herstellung einer monolithischen Arzneiform aus dem Granulat umfasst in Ausführungsformen, in denen die Arzneiform wenigstens zwei Kompartimente umfasst, bevorzugt das Verpressen der wenigstens zwei Kompartimentgranulate.

Es werden die Inhaltsstoffe des jeweiligen Kompartiments vorzugsweise gemischt und einer Schmelzgranulation unterzogen. Vorzugsweise weist die erfindungsgemäße Arzneiform keinen Überzug auf. In alternativen Ausführungsformen weist die Arzneiform einen Überzug auf, der gut löslich ist.

Bei der Herstellung der beiden Kompartimente, werden vorzugsweise die Bestandteile des ersten Kompartiments miteinander vermengt und ein Schmelzgranulat daraus hergestellt. Das gleiche wird vorzugsweise mit den Bestandteilen des zweiten Kompartiments durchgeführt. Es kann aber auch ein Granulat mit Hilfe eines Sprühverfahrens hergestellt werden.

Dann wird aus den Granulaten eine Arzneiform hergestellt. Im Falle einer Schichttablette wird das Granulat in einer Tablettenpresse verpresst.

In bevorzugten Ausführungsformen des Herstellungsverfahrens wird der erste Wirkstoff zur Herstellung des ersten Kompartimentes mit dem Emulgator vermengt. Vorzugsweise wird der Wirkstoff in dem Emulgator gelöst. Optional kann ein Lösungsmittel verwendet werden. Dann wird Wirkstoff, Emulgator und Lösemittel gemischt. Je nach genauer Beschaffenheit von Wirkstoff und Emulgator sind verschiedene Lösungsmittel denkbar. Vorzugsweise werden in der pharmazeutischen Technologie übliche Hilfsstoffe verwendet, insbesondere Alkohole, Ester und/oder Ketone, insbesondere Aceton.

Diese Mischung wird dann vorzugsweise auf einen Trägerstoff aufgesprüht. Bei dem Trägerstoff kann es sich um das oben beschriebene Presshilfsmittel und/oder das Verzögerungsmittel handeln. Das Aufsprühen kann vorzugsweise in einer Wirbelschichtanlage erfolgen.

In einer alternativen Ausführungsform wird der Wirkstoff mit dem Emulgator gemischt, insbesondere in einem Zwangsmischer, und geschmolzen. Diese Mischung wird dann auf den Trägerstoff aufgetragen, bei dem es sich um das Presshilfsmittel und/oder das Verzögerungsmittel handeln kann.

Die durch das Schmelzverfahren oder das alternative Sprühverfahren hergestellten Granulate können vor dem Verpressen mit Hilfsstoffen wie Gleitmitteln und Fließhilfsmitteln versetzt werden und dann direkt zu den gewünschten Kompartimenten verpresst werden.

Sofern erforderlich, wird der Wirkstoff zuvor zerkleinert, insbesondere in einer Nanomühle. Das so erhaltene Pulver wird vorzugsweise in den Emulgator eingelagert. Zur Einlagerung wird ein Lösungsmittel verwendet, in dem sich der Wirkstoff nicht löst, insbesondere Wasser. Die Einlagerung verhindert die Reagglomeration der Wirkstoffpartikel.

Das zweite Kompartiment kann auf gleiche Weise hergestellt werden. Vorzugsweise wird ein Granulat von Wirkstoff und Retardierungsmittel hergestellt, beispielsweise im Wege einer Feuchtgranulierung, dieses Granulat wird dann ggf. mit Hilfsstoffen zu dem zweiten Kompartiment verpresst.

### Beispiele

### Beispiel 1

Es wurden Arzneiformen der folgenden Zusammensetzungen hergestellt (Mengenangaben jeweils in mg):

| **1. Kompartiment** | **Ex.1** | **Ex.2** | **Ex.3** | **Zweck** |
|---|---|---|---|---|
| Cinnarizin | 60 | 60 | 60 | 1. Wirkstoff |
| Gelucire® 50/13 | 120 | 240 | 480 | Emulgator |
| Pharmatose | 480 | 480 | 480 | Presshilfsmittel |

Dabei wurden die angegebenen Inhaltsstoffe vermengt und dann im Wege einer üblichen Schmelzgranulation zu einem Granulat verarbeitet und anschließend zu Tabletten verpresst. Die Freisetzung wurde bei pH 5,5 mit einer Paddle-Apparatur ermittelt.

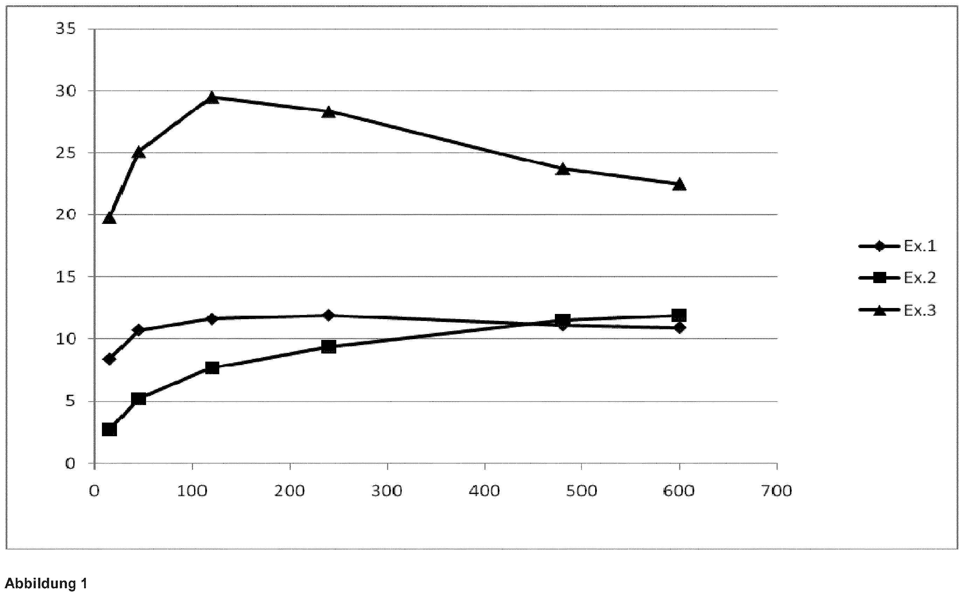

Die Abbildung 1 zeigt, welchen Einfluss die relative Menge an Emulgator an der Arzneiform auf das Lösungsverhalten hat. Die Ordinate zeigt die freigesetzte Wirkstoffmenge in Prozent, die Abszisse die vergangenen Minuten. Es ist erkennbar, dass die Menge an Emulgator auch die freigesetzte Wirkstoffmenge erhöht.

### Beispiel 2

Es wurde eine Arzneiform hergestellt, die folgende Zusammensetzung aufwies:

| **erstes Kompartiment** | **Menge in mg** | **Zweck** |
|---|---|---|
| Cinnarizin | 60 | 1. Wirkstoff |
| Gelucire® 50/13 | 240 | Emulgator |
| Dynasan® 118 | 240 | Triglycerid |
| Pharmatose | 480 | Presshilfsmittel |
| Summe | 1020 | |

| **zweites Kompartiment** | | |
|---|---|---|
| Dimenhydrinat | 120 | 2. Wirkstoff |
| Nacol 16-98 | 80 | langkettiger Alkohol |
| Methocel E4M (4000 cP) | 120 | Retardierungsmittel |
| Summe | 320 | |

Dabei wurden zunächst die Inhaltsstoffe des ersten Kompartiments miteinander vermischt und mittels Schmelzgranulation zu einem Granulat verarbeitet. Danach wurden auch die Inhaltsstoffe des zweiten Kompartiments in gleicher Weise zu einem Schmelzgranulat verarbeitet. Danach wurden beide Granulate zusammen in einer Tablettenpresse zu einer Arzneiform mit zwei Kompartimenten verpresst.

### Beispiel 3

Das folgende Beispiel zeigt den Einfluss eines Triglycerids auf die Wirkstofffreisetzung des schwerlöslichen Wirkstoffes. Der Versuch wurde bei einem pH-Wert von 5,5 in einer Paddle-Apparatur vorgenommen. Die Mengenangabe der Bestandteile erfolgt in mg.

| **1. Kompartiment** | **Ex.4** | **Ex.5** | **Zweck** |
|---|---|---|---|
| Cinnarizin | 60 | 60 | 1. Wirkstoff |
| Gelucire® 50/13 | 240 | 240 | Emulgator |
| Dynasan 118 | 0 | 240 | Triglycerid |
| Pharmatose | 480 | 480 | Presshilfsmittel |

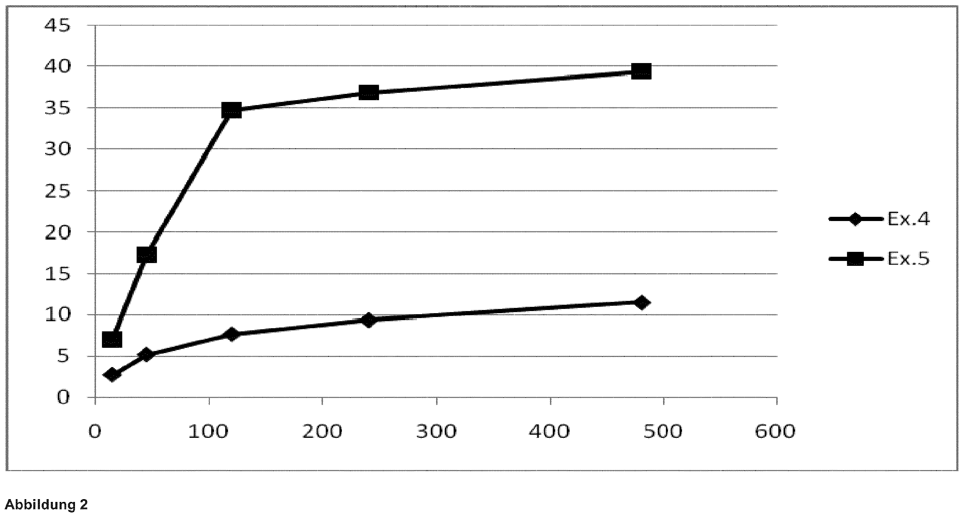

Die Abbildung 2 zeigt, welchen Einfluss das Triglycerid in der Arzneiform auf das Lösungsverhalten des ersten Wirkstoffes hat. Es ist erkennbar, dass das Triglycerid die Freisetzung von Cinnarizin verstärkt. So waren gemäß Ex.5 nach 480 min bereits 23,6 mg Cinnarizin in Lösung. Dies ist überraschend und vorteilhaft, weil sich Cinnarizin bei einem pH-Wert von 5,5 (wässriger Puffer) nur zu maximal 1 bis 2 mg löst. Die Ordinate zeigt die freigesetzte Wirkstoffmenge in Prozent, die Abszisse die vergangenen Minuten.

### Beispiel 4

Das folgende Beispiel zeigt weitere Versuche zur Ermittlung des Einflusses des Emulgators auf die Menge an gelöstem, schwer löslichem Wirkstoff. Hierfür wurden nachfolgende Zusammensetzungen zu Tabletten verpresst (Mengenangaben jeweils in mg). Die Prüfung erfolgte bei pH 5,5 in einer Bio-Dis-Anlage (30 Minuten mit 15 Dip/Minute). Jeweils nach 30 Minuten erfolgte ein Wechsel des Gefäßes. Die Lösungen wurden photometrisch ausgewertet.

| **1. Kompartiment** | **Ex.6** | **Ex.7** | **Zweck** |
|---|---|---|---|
| Cinnarizin | 20 | 60 | 1. Wirkstoff |
| Gelucire® 50/13 | 160 | 480 | Emulgator |
| Pharmatose, wasserfrei | 160 | 480 | Presshilfsmittel |

Es ergaben sich folgende Werte für die Menge an gelöstem Cinnarizin:

| **t [Minuten]** | **Ex.6** | **Ex.7** |
|---|---|---|
| 30 | 2,14 | 3,92 |
| 60 | 1,12 | 2,15 |
| 90 | 1,27 | 2,26 |
| 120 | 0,85 | 2,36 |
| 150 | - | 2,07 |
| 180 | 0,45 | 1,57 |
| **Gesamt:** | **5,83** | **14,33** |

Es ist überraschend, dass bei Erhöhung der Gesamtmenge an Wirkstoff auch die absolute Menge an Wirkstoff, die sich löst, erhöht ist. Damit wird deutlich, dass die Menge an freigesetztem Cinnarizin durch den Emulgator beeinflusst wird und nicht durch eine Grenzkonzentration beschränkt ist.

### Beispiel 5

Das folgende Beispiel zeigt Versuche zur Formulierung eines zweiten Kompartiments der erfindungsgemäßen Arzneiform. Dazu wurden mit den nachfolgenden Zusammensetzungen (Mengenangaben jeweils in mg) nach dem erfindungsgemäßen Herstellungsverfahren Schichttabletten hergestellt mit Abmessungen von 18x8 mm, wobei für das 1. Kompartiment eine Placebomischung verwendet wurde.

| **2. Kompartiment** | **Ex.8** | **Zweck** |
|---|---|---|
| Dimenhydrinat | 120 | 2. Wirkstoff |
| Nacol 16-98 | 80 | langkettiger Alkohol |
| Methocel E4M | 120 | Retard ierungsm ittel |

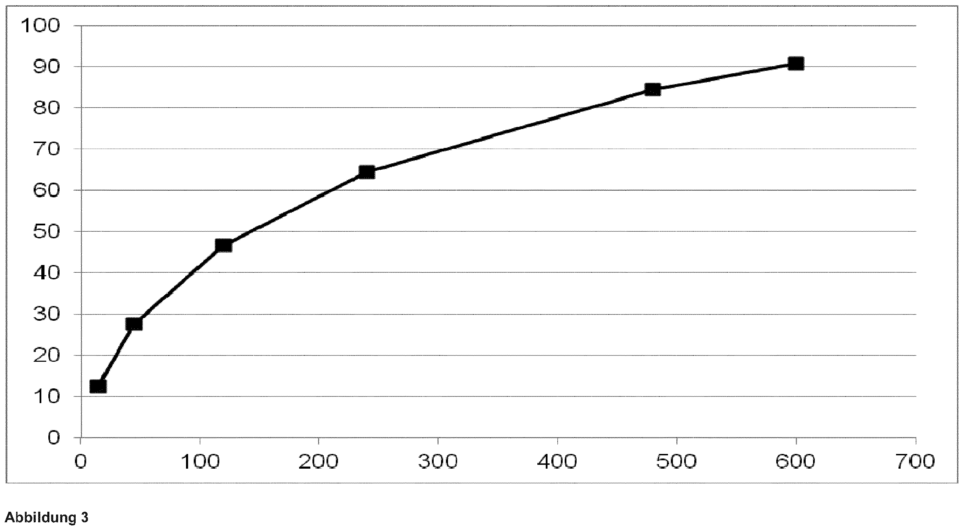

Die Abbildung 3 zeigt die Freisetzung von Dimenhydrinat aus der Arzneiform. Die Ordinate zeigt die freigesetzte Wirkstoffmenge in Prozent, die Abszisse die vergangenen Minuten. Es ist erkennbar, dass zunächst innerhalb von 60 Minuten Dimenhydrinat schnell aus dem 2. Kompartiment freigesetzt, wobei der Wirkstoff anschließend langsamer und nahezu linear freigesetzt wird. Es wurde eine Retardierung des Wirkstoffs im 2. Kompartiment über 10 Stunden erreicht.

## Patentansprüche

1. Arzneiform mit wenigstens einem ersten Wirkstoff und wenigstens einem Emulgator, wobei es sich bei dem Emulgator um Polyethylenglykolglyceride handelt, die durch Reaktion von Mono-, Di- und/oder Triglyceriden mit Polyethylenglykol hergestellt werden, wobei die Polyethylenglykolkette den hydrophilen Teil des Emulgators darstellt und mit einem lipophilen Teil verbunden ist, wobei der Emulgator einen HLB-Wert von wenigstens 1 und höchstens 16 aufweist,
wobei das Massenverhältnis von Wirkstoff zu Emulgator wenigstens 1 zu 30 und höchstens 1 zu 1 beträgt, und
wobei die Arzneiform eine Schichttablette ist, die wenigstens zwei Schichten aufweist, wobei die erste Schicht ein erstes Kompartiment ist und die zweite Schicht ein zweites Kompartiment ist,
wobei das erste Kompartiment wenigstens den ersten Wirkstoff und wenigstens den Emulgator umfasst und der Emulgator in dem zweiten Kompartiment nicht vorhanden ist,
wobei der erste Wirkstoff Cinnarizin oder ein Cinnarizinsalz ist.

2. Arzneiform nach Anspruch 1, wobei das Massenverhältnis von Wirkstoff zu Emulgator weniger als 1 zu 1 beträgt.

3. Arzneiform nach Anspruch 1, wobei die Kompartimente unterschiedliche Wirkstoffe enthalten.

4. Arzneiform nach wenigstens einem der vorhergehenden AnSprüche, wobei die Arzneiform in dem zweiten Kompartiment einen zweiten Wirkstoff enthält.

5. Arzneiform nach Anspruch 4, wobei der zweite Wirkstoff sich in Wasser in einer Menge von mehr als 0,5 Mol/l löst.

6. Arzneiform nach Anspruch 5, wobei ein Retardierungsmittel im zweiten Kompartiment enthalten ist.

7. Arzneiform nach Anspruch 5 oder 6 , wobei der zweite Wirkstoff Dimenhydrinat ist.

8. Arzneiform nach wenigstens einem der vorhergehenden Ansprüche, wobei die Arzneiform eine Schichttablette in Form einer Mantel-Kern-Tablette ist.

9. Verfahren zur Herstellung einer Arzneiform nach wenigstens einem der vorhergehenden Ansprüche mit den Schritten:
a. Mischen der Bestandteile der Arzneiform,
b. Granulieren der Mischung und
c. Herstellung einer monolithischen Arzneiform aus dem Granulat.

10. Arzneiform nach einem der Ansprüche 1 bis 8 zur Verwendung in einem Therapieverfahren.

## Claims

1. A pharmaceutical form with at least one first active substance and at least one emulsifier, wherein the emulsifier is polyethylene glycol glycerides which are prepared by reaction of mono-, di- and/or triglycerides with polyethylene glycol, wherein the polyethylene glycol chain is the hydrophilic part of the emulsifier and is connected with a lipophilic part, wherein the emulsifier has an HLB value of at least 1 and at most 16, wherein the mass ratio of active substance to emulsifier is at least 1 to 30 and at most 1 to 1, and
wherein the pharmaceutical form is a layer tablet comprising at least two layers, wherein the first layer is a first compartment and the second layer is a second compartment, wherein the first compartment comprises at least the first active substance and at least the emulsifier, and the emulsifier is not present in the second compartment,
wherein the first active substance is cinnarizine or a salt of cinnarizine.

2. The pharmaceutical form according to claim 1, wherein the mass ratio of active substance to emulsifier is lower than 1:1.

3. The pharmaceutical form according to claim 1, wherein the compartments contain different active substances.

4. The pharmaceutical form according to at least one of the preceding claims, wherein the pharmaceutical form contains a second active substance in the second compartment.

5. The pharmaceutical form according to claim 4, wherein the second active substance dissolves in water in an amount of more than 0.5 mol/l.

6. The pharmaceutical form according to claim 5, wherein a retarding agent is contained in the second compartment.

7. The pharmaceutical form according to claim 5 or 6, wherein the second active substance is dimenhydrinate.

8. The pharmaceutical form according to at least one of the preceding claims, wherein the pharmaceutical form is a layer tablet in the form of a coat-core-tablet.

9. A method for the production of a pharmaceutical form according to at least one of the preceding claims with the steps:
a. mixing the constituents of the pharmaceutical form,
b. granulating the mixture and
c. preparing a monolithic pharmaceutical form from the granules.

10. The pharmaceutical form according to one of claims 1 to 8 for use in a therapy method.

## Revendications

1. Forme galénique avec au moins une première substance active et au moins un agent émulsifiant, dans laquelle l'agent émulsifiant est du glycéride de polyéthylèneglycol, qui est produit par la réaction de mono-, di- et/ou triglycéride avec du polyéthylèneglycol, dans laquelle la chaîne de polyéthylèneglycol constitue la partie hydrophile de l'agent émulsifiant et est liée avec une partie lipophile, dans laquelle l'agent émulsifiant présente un indice HLB d'au moins 1 et d'au plus 16,
dans laquelle le rapport de masse entre la substance active et l'agent émulsifiant est d'au moins 1 sur 30 et d'au plus 1 sur 1, et
dans laquelle la forme galénique est un comprimé en couches, qui présente au moins deux couches, dans laquelle la première couche est un premier compartiment et la deuxième couche est un second compartiment,
dans laquelle le premier compartiment comprend au moins la première substance active et au moins l'agent émulsifiant et l'agent émulsifiant n'est pas présent dans le second compartiment,
dans laquelle la première substance active est de la cinnarizine ou un sel de cinnarizine.

2. Forme galénique selon la revendication 1, dans laquelle le rapport de masse entre la substance active et l'agent émulsifiant est inférieur à 1 sur 1.

3. Forme galénique selon la revendication 1, dans laquelle le compartiment contient différentes substances actives.

4. Forme galénique selon au moins l'une quelconque des revendications précédentes, dans laquelle la forme galénique dans le second compartiment contient une seconde substance active.

5. Forme galénique selon la revendication 4, dans laquelle la seconde substance active se dissout dans de l'eau selon une quantité de plus de 0,5 mole/l.

6. Forme galénique selon la revendication 5, dans laquelle un agent retardateur est présent dans le second compartiment.

7. Forme galénique selon la revendication 5 ou 6, dans laquelle la seconde substance active est un diménhydrinate.

8. Forme galénique selon au moins l'une quelconque des revendications précédentes, dans laquelle la forme galénique est un comprimé en couches sous la forme d'un comprimé gaine/cœur.

9. Procédé de production d'une forme galénique selon au moins l'une quelconque des revendications précédentes comprenant les étapes de :
a. mélange des composants de la forme galénique,
b. granulation du mélange et
c. production d'une forme galénique monolithique à partir du granulat.

10. Forme galénique selon l'une quelconque des revendications 1 à 8 pour une utilisation dans un procédé de traitement.
